(19) **Europäisches Patentamt** / **European Patent Office** / **Office européen des brevets**

(11) **EP 4 537 861 A1**

(12) **EUROPEAN PATENT APPLICATION**

(43) Date of publication:
**16.04.2025 Bulletin 2025/16**

(21) Application number: **23203649.1**

(22) Date of filing: **13.10.2023**

(51) International Patent Classification (IPC):
**A61L 27/18** (2006.01)    **C08G 71/04** (2006.01)

(52) Cooperative Patent Classification (CPC):
(C-Sets available)
**A61L 27/18; C08G 18/4288; C08G 18/7671;**
A61L 2430/02; A61L 2430/04      (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **b2bond GmbH**
**67549 Worms (DE)**

(72) Inventors:
• **DE AZEVEDO, Paulo**
**36773-016 Cataguases (BR)**
• **ROSSI, Antonio Carlos**
**17207-690 Jaú (BR)**

(74) Representative: **Prüfer & Partner mbB**
**Patentanwälte · Rechtsanwälte**
**Sohnckestraße 12**
**81479 München (DE)**

(54) **BIOCOMPATIBLE POLYURETHANE MATRIX**

(57) The present invention refers to a biocompatible polyurethane (PU) matrix comprising essentially no water, and wherein free fatty acid is present but the content thereof is up to 19 %, as well as to a process for preparing the PU matrix, wherein this process comprises a step of providing a polyol component and a prepolymer component. The present invention further refers to the PU matrix for use in bone-related methods, as well as to a kit that comprises the polyol component and the prepolymer component.

Fig. 1

**(Cont. next page)**

(52) Cooperative Patent Classification (CPC): (Cont.)

C-Sets
**A61L 27/18, C08L 75/04;**
**C08G 18/10, C08G 18/42**

**Description**

Field of the invention

The present invention refers to a biocompatible polyurethane (PU) matrix comprising essentially no water, and wherein the content of free fatty acid is limited, as well as to a process for preparing the PU matrix, wherein this process comprises a step of providing a polyol component and a prepolymer component. The present invention further refers to the PU matrix for use in bone-related methods, as well as to a kit that comprises the polyol component and the prepolymer component.

Description of the background art

The treatment of undesired bone conditions or bone defects, such as bone fractures or loss of bone, has a long-lasting history in medicine. For example, bone fractures are treated by conservative methods such as stabilization through plaster or surgical procedures by application of external or internal fixatures, e.g., screws, metal plates or wires.

On the other hand, bony defects are covered by insertion of metal or plastic plates and/or bone material derived from the same organism. Comparable procedures are used for implants, such as dental implants or orthopaedic implants; these implants require a stable insertion e.g., by cement or glues into bone tissue to exert the necessary strength.

The major disadvantage of these procedures is the long-lasting healing process which is completely dependent on the individual bone regenerative potential of the bone and its specific integration capability and fixation of foreign material to or in the bony tissue. The natural healing process often requires a longer lasting period of immobilization with all its individual and economic consequences and disadvantages before the natural cascade of osseo-regeneration process leads to the necessary mechanical stability.

Dental and medical implants most frequently made from titanium or hipped zirkonoxides have a significant rate of failure either due to infection or loosening of the implant.

Many methods are described in prior art and partially brought to practice to overcome these limitations and ensure a stable connection between foreign material and the bone or shorten the period before e.g., a fracture can be exposed to mechanical force. However, the materials described so far in prior art are having various disadvantages, for example there is no bone glue currently marketed which could functionally fix and stabilize instantly broken bones; further, bone cements used for anchoring implants in orthopaedic medicine are hardening in a strong exothermic reaction causing necrosis of vital osteoblasts in the bone - implant - interface. Such endoprostheses often have to be removed after the bone is stabilized, thereby requiring a second surgical procedure. Further, previous materials marketed for covering bone defects were taken from market due to the unpredictable hardening time and variable volume change during the hardening process and thereafter.

Hatt et al. further discloses some of the requirements an implant desirably has to meet.

Hence, there is a need for and thus it is an object of the present invention to provide an improved material which overcomes at least one, preferably all, of the above disadvantages. Thus, advantageously, an improved material is suitable for being used in treating bone defects and/or undesired bone conditions, and exhibits one or more, preferably all, of the following properties: The improved material should be flexibly formed *ex vivo* and *in vivo* in order to obtain the desired shape; it should be able to provide comparably high gluing properties which e.g., allows a rapid exposure to mechanical force; it should have predictable properties for hardening; during the hardening (curing/polymerization) process, there should be no temperature increase *in vivo* which harms the affected site; the improved material should be highly biocompatible with living tissue, particularly with bone, without causing local inflammation and toxicity and it should integrate with the bone tissue; the improved material should show osteoconduction, that is, attraction and natural incorporation of bone cells such as mesenchymal stem cells, osteointegrin expressing cells, and osteoblasts, into the material thereby resulting in gradual replacement of the implant/glue interface by natural bone; it should prevent bacterial growth and formation of biofilms; and/or the improved material should be able to be loaded with substances such as calcium-salts (e.g., calcium carbonate, or calcium phosphate), peptides, proteins, and other materials that have osteopromotional and pH regulating properties. Moreover, the PU matrix advantageously should provide for a surface that does not support the colonialization of the surface with activated macrophages, which play an important role in induction of inflammation.

It is further an object of the present invention to provide a method for producing such material, and to provide a kit which provides components of the improved material.

These as well as further objects, which will become apparent from the following description of the present invention, are attained by the subject matter of the independent claims. Some of the preferred embodiments of the present invention are defined by the subject matter of the dependent claims.

Summary of the invention

Various aspects, advantageous features and preferred embodiments of the present invention as summarized in the following items, respectively alone or in combination, contribute to solving the object of the invention.

(1) Polyurethane (PU) matrix, wherein

i) a water content of the PU matrix is equal to or less than 1%, preferably equal to or less than 0.1%, more preferably there is no water present, as determined by moisture content measurement method (oven dry method); and/or
ii) free fatty acid is present but the content thereof is up to 19%, preferably between 7% and 19%, more preferably between 5% and 15%, and most preferably between 7% and 10%, as determined by titration with sodium hydroxide,

with the respective properties determined at a temperature of 25°C.
In a preferred embodiment, the source of the free fatty acid that is present in the PU matrix is derived from castor oil. Castor oil is derived from up to 93% (about 81%-93%) ricinoleic acid, and about 7% to 19% remaining acids (i.e., further components). These further components are oleic acid (about 2%-8%), linoleic acid (about 1 %-6%), alpha-linolenic acid (about 0.5%-2%), stearic acid (about 0.5%-1 %), palmitic acid (about 0.5%-1 %), dihydroxystearic acid (about 0.3% -0.5%) and other components (about 0.2%-0.5%).
In a particular preferred embodiment, the source of free fatty acid is the fatty acids that are derived from the castor oil, but not or only to a limited extent ricinoleic acid. Hence, preferably, the source of free fatty acid is oleic acid, linoleic acid, alpha-linolenic acid, stearic acid, palmitic acid, dihydroxystearic acid, and/or other components. Consequently, in a particular preferred embodiment, the content of free fatty acid (excluding ricinoleic acid) in the PU matrix is up to 19%, preferably between 7% and 19%, and the content of ricinoleic acid-derived component in the PU matrix is 81% or more. In other words, the content of ricinoleic acid - derived component in the PU matrix is 81% or more, and the remaining free fatty acid content is up to 19%.

(2) PU matrix according to (1), wherein the matrix additionally exhibits one or more, preferably all, of the following properties:

iii) physical resistance capacity against traction being in the range of between 26 MPa and 42 MPa; preferably being in the range of between 30 MPa and 38 MPa, as determined according American Society of Testing and Materials ASTM D695MM;
iv) elastic modulus being in the range of between 1700 Mpa and 2400 Mpa, preferably being in the range of between 1900 Mpa and 2200 Mpa, as determined according to American Society of Testing and Materials ASTM D695MM;
v) compression being in the range of between 48 Mpa and 57 Mpa, preferably in the range of between 50 Mpa and 55 Mpa, as determined according to American Society of Testing and Materials ASTM D695MM;
vi) deformation being in the range of between 3.7% and 5.5%, preferably in the range of between 4.0% and 5.0%, as determined according to American Society of Testing and Materials ASTM D695MM; and
vii) hardness shore D being in the range between 72 and 84, preferably between 75 and 80, as determined by a durometer according to American Standards for Testing and Materials ASTM D2240,

with the respective properties determined at a temperature of 25°C

(3) PU matrix according to (1) or (2), additionally comprising further compounds being selected from the group consisting of Ca-salts; hydroxylapatite; small molecules, peptides or proteins having bone inducing properties; and small molecules, peptides, or proteins having antiinfective and/or antibacterial properties.

(4) PU matrix according to any one of the preceding items, being in form of a liquid or a solid.

(5) PU matrix according to any one of the preceding items, being in form of a solid biocompatible flexible sheet and being thermoformable into changed shape by being deformed under heat, without elastic memory and return into original shape, preferably the solid biocompatible flexible sheet having a thickness in the range of from equal to or more than 0.01mm to equal to or less than 20.00mm.

(6) PU matrix according to item (5), wherein

- the PU matrix having a thickness being in the range of from 0.01mm to less than 1.00mm being defined as a membrane;
- the PU matrix having a thickness being in the range of from 1.00mm to less than 5.00mm being defined as a blade; and
- the PU matrix having a thickness being in the range of from 5.00mm to less than 20.00mm being defined as a block.

(7) Process for preparing a polyurethane matrix, in particular for preparing the polyurethane matrix, preferably the matrix as defined in any of items (1) to (6), comprising the following steps:

a) providing a polyol component (also referred to herein as "Ampoule A"), wherein the polyol component has:

(a-i) an acidity index being in the range of from 0.8-8.5 mgKOH/g, preferably from 1.0-7.5 mgKOH/g, more preferably from 1.5 to 7.0 mgKOH/g, even more preferably from 2.5-6.5 mgKOH/g, even more preferably from 4.0-5.5 mgKOH/g, and most preferably from 4.5-5.0 mgKOH/g, as determined by titration;
(a-ii) a hydroxyl index in the range of from 200 to 450 mgKOH/g preferably from 250 to 400 mgKOH/g , more preferably from 300 to 400 mgKOH/g, and most preferably from 360 to 380, as determined by the method disclosed in "The International Pharmacopoeia - Eleventh Edition, 2022, 4.7 Determination of hydroxyl value"; and

b) providing a diisocyanate component, preferably diphenylmethane-4,4'-diisocyanate (MDI), wherein the diisocyanate component has:

(b-i) an amount of free isocyanate as NCO equivalent being in the range of from 16% to 35% preferably from 22% to 35%, more preferably from 25% to 32% as determined by addition of excess di-n-butyl amine to form a urea and subsequent back titration with hydrochloric acid of the unreacted amine,
(b-ii) optionally a density being in the range of from 1.11 to 1.28 g/cm$^3$, as determined by pycnometry;

c) combining a at least a part of the polyol component of a) and at least a part of the diisocyanate component of b) to form a prepolymer (also referred to herein as "Ampoule B");

d) combining at least a part of the polyol component of a) and at least a part of the prepolymer of c), thereby preparing a mixture; and

e) obtaining the polyurethane matrix from said mixture.

(8) Process according to item (7), wherein
the prepolymer of step c) has

(c-i) an amount of free isocyanate as NCO equivalent being in the range of from 10% to 25%, preferably from 19% to 23%, more preferably from 20 to 21%, as determined by addition of excess di-n-butyl amine to form a urea and subsequent back titration with hydrochloric acid of the unreacted amine;
(c-ii) a density being in the range of from 1000 to 1500 g/cm$^3$, preferably from 1100 to 1400 g/cm$^3$, more preferably from 1180 to 1270 g/cm$^3$, as determined by pycnometry at a temperature of 25°C; and/or
(c-iii) a dynamic viscosity being in the range from 350-750 centipoise (cP), preferably from 400 to 700, more preferably from 450 to 650 cP, and even more preferably from 500 to 600 cP, as determined with a rotational viscometer by ASTM D4878.

(9) Process according to item (7) or item (8), wherein

a) the polyol component (a) additionally has

(a-iii) a density being in the range from 850-1200 g/cm$^3$, preferably from 950 to 1200 g/cm$^3$, more preferably from 1000 to 1150 g/cm$^3$ , and most preferably from 1050 to 1100 g/cm$^3$, respectively as determined by pycnometry at a temperature of 25°C; and
(a-iv) a viscosity being in the range from 1400 to 1800 cP, preferably from 1500 to 1700 cP, as determined by ASTM D4878; and/or

b) the diisocyanate component (b) additionally has

(b-iii) a water content of equal to or less than 0.1 %, preferably being in the range from 0.01 % to 0.1 %, more preferably the water content is zero (0%), as determined by moisture content measurement (oven dry method); and

(b-iv) a viscosity being in the range from 20 cP to 200 cP, preferably being in the range from 20 cP to 120 cP, more preferably being in the range from 40 cP to 120 cP, and even more preferably being in the range from 40 cP to 100 cP, as determined by ASTM D4878.

(10) Process according to any one of items (7) to (9), wherein
the polyol component of step a) is obtained from castor oil, preferably from chemically reacted castor oil, more preferably wherein the original castor oil used has one or more, in particular all of the following characteristics:

o an acidity index being in the range of from 0.20-1.5 mgKOH/g, preferably from 0.40-1.25 mgKOH/g, more preferably from 0.5 to 1.0 mgKOH/g as determined by titration using potassium hydroxide;
o a hydroxyl index in the range of from 150-170, preferably from 150-160, more preferably from 155 to 165, as determined by the method disclosed in "The International Pharmacopoeia - Eleventh Edition, 2022, 4.7 Determination of hydroxyl value"; and optionally
o a density being in the range from 0.090-0.098 g/cm$^3$, preferably from 0.095-0.096 g/cm$^3$, as determined by pycnometry at a temperature of 25°C; and/or
o a viscosity being in the range from 500 cP to 1000 cP, preferably being in the range from 600 cP to 900 cP, more preferably being in the range from 700 cP to 800 cP, as determined by ASTM D4878.

(11) Process according to any one of items (7) to (10), wherein in step c) the following parts of polyol component and diisocyanate component are combined: 1 part of polyol and 1 to 1.7 part of diisocyanate; preferably 1 part of polyol to 1.2 to 1.6 part of diisocyanate, and more preferably 1 part of polyol to 1.3 to 1.5 part of diisocyanate.
In a preferred embodiment, by combining the parts of polyol component and diisocyanate component as indicated above, the diisocyanate component is present in the prepolymer in an amount of 20% to 32%.

(12) Process according to any one of items (7) to (11), wherein in step d), the ratio of the prepolymer of step c) that is combined with the polyol of step a) to obtain a polyurethane mixture is in a range of from 0.5:1.0 to 0.8:1.0 (polyol/prepolymer).

(13) Process according to any one of items (7) to (12), wherein step a) of providing a polyol component comprises the following steps:

(a-1) combining castor oil, ethylene glycol and propylene glycol under protective atmosphere, preferably nitrogen atmosphere; preferably, the combining is carried out under stirring; preferably for a time period of 1 to 5 hours, more preferably of 2 to 4 hours, even more preferably for about 2 hours; thereby arriving at a mixture;
(a-2) heating up the mixture of (a-1), preferably heating up to a temperature in the range of from about 100°C to about 270°C, more preferably to a temperature in the range from about 200°C to about 260°C, even more preferably to a temperature of about 250°C, followed by a step of cooling;
(a-3) optionally rotating the mixture, preferably under reduced pressure, preferably in a rotary evaporator, thereby eliminating residual gas;
(a-4) optionally cooling the mixture; and
(a-5) obtaining the polyol component.

(14) Process according to any one of items (7) to (13), wherein step c) of forming a prepolymer comprises the following steps:

(c-1) mixing the polyol of step (a) with a diisocyanate compound (e.g. diphenylmethane-4,4"-diisocyanate (MDI)), preferably in a ratio as defined above, thereby obtaining a mixture;
(c-2) stirring the mixture of (c-1), preferably at a temperature of about 100°C;
(c-3) optionally applying a vacuum;
(c-4) removing possible gas inclusion, preferably by evaporating the mixture; and
(c-5) obtaining the prepolymer.

(15) Process according to any one of items (7) to (14), comprising a further step of adding further compounds, wherein the further compounds are selected from the group consisting of

- Ca-salts such as $CaCO_3$ or $CaCO_3(PO_4)_2$, wherein preferably these salts are present in a total amount of 10wt% to 55wt%, preferably 20wt% to 50wt%, more preferably 30wt% to 50wt%, even more preferably 30wt% to 45wt%, and most preferred in a total amount of 30wt% to 40wt%, based on the sum of the weight of the main components polyol and prepolymer;
- hydroxylapatite, wherein preferably the hydroxylapatite is present in an amount of about 15-25wt%, more preferably in an amount of 18-22wt.%, and most preferably in an amount of about 20wt.%, based on the sum of the weight of the main components polyol and prepolymer;
- small molecules, peptides, or proteins having bone inducing properties, wherein preferably the total amount of said components is in a range of from about 1wt% to about 5wt%, based on the sum of the weight of the main components polyol and prepolymer; and
- small molecules, peptides, or proteins having antiinfective and/or antibacterial properties, in a suitable amount.

(16) Process according to item (15), wherein the further compound that is added in a further step is a Ca-salt such as $CaCO_3$ or $CaCO_3(PO_4)_2$, preferably the further compound that is added is $CaCO_3$.

(17) Process according to any one of items (7) to (16), wherein the further compound is added during or after step d). In a preferred embodiment, the further compound is added during or after step d), but prior to step e). Preferably, the further compound is added during step d), thereby being part of the mixture of step d).

(18) Process according to any one of items (7) to (17), wherein step e) comprises a step of polymerizing the mixture of step d).

(19) Process according to any one of items (7) to (18), wherein the maximum polymerization temperature that is developed during the polymerizing step is at most 47°C, preferably at most 46°C, or at most 45°C, when measured inside a container where step d) takes place.
In a preferred embodiment, when the mixture is applied to the bone, the temperature of this mixture is less than 47°C, preferably the temperature is 45°C or less, more preferably, the temperature is 43°C or less.

(20) Process according to any one of items (7) to (19), wherein during step e) no polymerization catalyst is present.

(21) PU matrix, obtainable by the process as defined in any one of items (7) to (20).

(22) The PU matrix of any one of items (1) to (7), or according to item (21), for use in a method of therapeutic treatment for bone regeneration, bone fixation, bone adhesion ("glue"), bone implantation, and/or substitution of a whole or a part of a bone.

(23) The PU matrix for use according to item (22), wherein the method of treatment comprises preparing implants, preferably for 3D bone reconstruction and bone tissue engineering, such as dental implants or orthopaedic implants, preferably said implants are individualized implants, such as orthopaedic or dental individualized implants; preferably for use in preparing dental implants, more preferably for use in preparing individualized dental implants.

(24) The PU matrix for use according to item (22) or (23), wherein the implants are suitable for replacing bones; for replacing parts of bones; for replacing bone defects; or for stabilizing bone structures.

(25) The PU matrix for use according to any one of items (22) to (24), wherein the PU matrix is provided to a subject subcutaneously.

(26) A kit comprising the polyol component as defined in any one of items (7) to (10), and the prepolymer as defined in items (7), (8), (11), or (14).

(27) The kit according to item (25) or (26), further comprising one or more of the compounds selected from the group consisting of Ca-salts, hydroxylapatite, and small molecules, peptides, or proteins.

Brief description of the drawings

Fig. 1 shows shows various examples - e.g., implant forms of the final polymer such as dental membranes for guided tissue regeneration (GTR), spinal fusion Insert, plates, screws, buttons, granulated bone graft, bone segment or total bone prosthetic customized by prototyping.

Fig. 2 shows the bending stress of glued mini pig femur. It can be seen that glued bone has a comparably high strength.

Fig. 3 shows the strength of glued bone.

Fig. 4 shows a temperature curve of polyol as described in Example 1 and the prepolymer as described in Example 2 upon being mixed and stirred during the following 15 min until viscosity is achieved. As can be seen, the temperature peaks at 46°C after 13 minutes.

Detailed description of the invention

The present invention is now described in more detail by preferred embodiments and examples, which are however presented for illustrative purposes only and shall not be understood as limiting the scope of the present invention in any way.

Polymers based on castor oil as starting material were described in the past for preparation of implants. Castor oil can be transformed into defined polyols that serve as a central component of the polymer together with e.g., diphenylmethane-4,4'-diisocyanate (MDI). The transformation of the castor oil into defined polyols can be carried out by certain processes, such as the processes described below. In addition, the polymer can be augmented (supplemented) with various ingredients, e.g., with ingredients supporting the bone formation.

The preparation of polyols from castor oil is based on the unique content of ricinoleic acid within the castor oil. Generally, castor oil is known as a source of ricinoleic acid, as it consists of up to 93% (about 81 %-93%) ricinoleic acid. Further components are oleic acid (about 2%-8%), linoleic acid (about 1%-6%), alpha-linolenic acid (about 0.5%-2%), stearic acid (about 0.5%-1%), palmitic acid (about 0.5%-1 %), dihydroxystearic acid (about 0.3% -0.5%) and other components (about 0.2%-0.5%). Polyfunctional polyesters are prepared and produced worldwide, however they are usually not made for use e.g., as a medical device in vivo due to its low biocompatibility. These polyurethanes, which are usually not suitable for being used in the medicinal field, are in most of the cases polymerized by the reaction of a polyol (polyfunctional alcohol), synthesized from the castor oil and an isocyanate group, being the MDI or TDI (toluene diisocyanate). The reaction occurs between molecules of isocyanate and the hydroxyl-moiety; it is assumed that in this setting, a remaining fraction of isocyanate may not react with the hydroxyl groups of the polyols, but rather immediately react with the remnant molecules of water within the system. This will generate carbamic acid, which decomposes and forms the primary amine and $CO_2$. The primary amine can also react with the isocyanate to form disubstituted urea. These reaction products are a determining factor for the observed low biocompatibility.

Some of these castor oil polymers were studied in the past with regard to their suitability for being used in the medical field, e.g., with regard to their suitability as implants, in animal studies.

In 1959, Mandarino and Salvatore implanted a rigid polyurethane oil foam into the bone, but it did not show the desired stabilizing or bone growth effects.

It has been found that a critical step in the use of castor oil in the field of medicine is the derivatization of the oil to polyols.

In the past, castor oil polymers were shown to react with TDI and MDI and were tested in the past for possible use in the medical field, specifically as bone replacement. However, the materials did not show the desired profile that would make it suitable for this specific field, as it showed disadvantageous properties, e.g., signs of toxicity, no elasticity for plastic deformation, and/or low strength (US Patent application 2012/0265312 A1, Norton et al. 2020, Doumit et al. 2014).

Another method for producing the castor oil polymers is propoxylation and ethoxylation, using catalysts like double metal cyanides (DMCs) (Musik et al. 2022; Valvekens and DeVos 2016). DMC are sufficiently catalyzing the reaction of castor oil with propylene oxide and ethylene oxide. However, since the catalysts cannot be removed they bear the risk of toxicity and decreased bioavailability.

Another method is epoxidation, subsequently used as a monomer in the synthesis of castor oil polymer (Hernandez et al. 2017). The epoxidation process is performed using a catalyst system of $H_2O_2$, $Al_2O_3$ and ethyl acetate). However, the use of metallic and aluminum-based catalysts are potentially cytotoxic or otherwise impair biocompatibility.

Another example is the synthesis under $N_2$ atmosphere, using the castor oil, toluene diisocyanate (TDI), N-dimethylformamide, p-toluene sulfonic acid and polyaniline.

Polyols can also be synthesized from chemical glycerolysis between castor oil and three different glycerins (Zhang et al. 2021), mainly for the purpose of coating of various materials.

In 1999, a polyurethane derived from the castor oil was developed, by the Analytical Chemistry Group of the University of São Paulo, Brazil, synthesized and commercialized (Polyqil) (Sousa et al. 2018, Beloti et al. 2003). No data are available how the polymer was prepared. However, the material was withdrawn from the market by the Brazilian Health Agency (ANVISA) obviously by missing biocompatibility and/or efficacy.

Another example of a castor oil polyurethane disclosed as a U.S. Patent titled "Polyurethanes of Improved Characteristics Biocompatible Urea-Modified Polyurethane from Castor Oil", No. 4,990,586, dated February 5 of 1991, in which the use of diamines to produce such polymers is disclosed. There are no products described which have ever brought into clinical studies or practice.

The previously described polymers contain free isocyanate molecules after polymerization, which have a strong reaction with residual water molecules and thus forming biurets. These reactions interfere with osseointegration and biocompatibility. Another disadvantage is the need of catalysts to foster the formation of the polymer.

The most advanced polymer used for bone implants was a polymer named "Kryptonite" from Doctors Research Group, which was developed in the early 2000. The material was allowed by FDA for cranioplastic surgery. The material consists of castor oil derived fatty acids, triglyceride being synthesized with NCO (isocyanate) groups to create pre-polymer chains ready for polymerization upon mixing with a second component also containing a naturally occurring hydroxyl terminated fatty acid derived from castor oil. In additional catalyst and water are used, reacting with the NCO groups to release carbon dioxide to produce porosity in the final material. A third component was calcium carbonate, a non-reactive filler to enhance porosity and assists in achieving the desired mechanical and structural properties (US 2012/0265312 A1). The main disadvantages were (i) the volume change observed after insertion of the implant (Doumit et al. 2014) which led to fatal disease courses after use in spine surgery; eventually the product was withdrawn from market due to FDA decision (Doumit et al. 2014).

Polyurethanes of petroleum, with limited biocompatibility, were obtained with the use of urethane and urea bonds. Transparent polyurethanes using aliphatic diisocyanate are for building artificial organs can also promote foreign body reaction. Castor oil modified by transesterification, using e.g., diethanolamide are leading to polyurethan foam with limited strength (Palanisamy et al. 2011).

In the present invention, a polyurethane (PU) is provided that overcomes one or more, preferably all, of the above-described disadvantages, and a method for preparing the same is also provided.

The invention herein discloses a novel polyurethan matrix and polyurethane polymer which surprisingly encompass the above-mentioned desired characteristics and overcome problems described for conventional polyurethan matrices and polymers. It has been found in the present invention that it is the controlled content of the free fatty acid and control of the water content as disclosed herein that contributes to obtaining a surprisingly improved PU matrix. Further, it has been found in the present invention that it is a combination of components exhibiting specifically selected characteristics and properties as disclosed herein which results in an obtaining such an improved PU matrix.

The PU matrix of the present invention is thus particularly valuable for being used in the medical field, for example as bone glue and/or as implantable material, which overcomes one or more of the disadvantages of the prior art products, such as in terms of biocompatibility problems. In this regard, the PU matrix of the present invention can further avoid the use of and/or the generation of possible harmful catalysts such as metallic catalysts, diamines, and presence of residual water. The polymer matrix of the present invention preferably has a high biocompatibility after implantation due to its chemical characteristics and also due to the unparalleled effect that the polymerization and hardening happens at a comparably low temperature (preferably such as equal to or less than 47°C, or equal to or less than 42°C) thus amongst others avoiding persistent inflammation and necrosis specifically at the site of implantation. It is strongly integrating to the bone surface, which is called osseointegration. This intimate "sticky" interaction with bone tissue results in a strong adhesion between two bone surfaces, with the PU matrix of the present invention having the function of a "glue", regardless of whether it is used in liquid form or introduced into organisms as solid bone grafts form, solid implants or solid prostheses.

The PU matrix of the present invention can also be used in cosmetic field, for example it is useful in the treatment of undesired cosmetic appearances of the skin, such as wrinkles. It is possible to separate and/or distinguish a cosmetic use from a therapeutic use, depending on the situation or desired use.

Thus, the present invention refers to a PU matrix wherein the water content is equal to or less than 1%, preferably equal to or less than 0.1 %, more preferably there is no water present, as determined by moisture content method; and/or wherein the content of free fatty acid is present but is controlled to be up to 19%, preferably between 7% and 19%, more preferably between 5% and 15%, and most preferably between 7% and 10% , as determined by titration with sodium hydroxide, with the respective properties determined at a temperature of 25°C. In a preferred embodiment, the PU matrix exhibits the above defined water content and free fatty acid content.

The PU matrix exhibiting the above water content and/or free fatty acid is improved with respect to one or more, preferably more, or even all, properties disclosed herein. For example, it exhibits improved biocompatibility, less toxicity e.g., because no catalysts are necessary, improved adhesion function ("glue"), improved plasticity and improved handling. The PU matrix of the present invention is particularly advantageous as it does not exhibit a temperature above 47°C during the polymerization and/or hardening process. Additionally, the inventive PU matrix is biocompatible and exhibits osseointegrative properties, which means that it is able to undergo gradual and slow absorption when it is contacted with bone (such as the defect bone that is to be repaired, or the bone that is to be glued), since a part of the PU matrix is composed of fatty acid that exists in the living organism recipient and that is replaced by new bone.

In a preferred embodiment, the PU matrix of the present invention in addition to the above-listed properties, exhibits one or more, preferably all, of the following properties as determined according to American Society of Testing and Materials ASTM D695 , Standard Test Method for Compressive Properties of Rigid Plastics, Volume 8.01, DOI: 10.1520/D0695-15 valid at the effective date: The physical resistance capacity against traction is in the range of between 26 MPa and 42 MPa; preferably in the range of between 30 MPa and 38 MPa; the elastic modulus is in the range of between 1700 Mpa and 2400 Mpa, preferably in the range of between 1900 Mpa and 2200 Mpa,; the compression is in the range of between 48 Mpa and 57 Mpa, preferably in the range of between 50 Mpa and 55 Mpa; the deformation is in the range of between 3.7% and 5.5%, preferably in the range of between 4.0% and 5.0%, as determined by ASTM;D695MM; and the hardness shore D is in the range between 72 and 84, preferably between 75 and 80, as determined by a durometer according to American Standards for Testing and Materials ASTM D2240 Standard Test Method for Rubber Property-Durometer Hardness, Vol. 0.01 DOI: 10.1520/D2240-15R21 valid at the effective date.. The respective properties are determined at a temperature of 25°C.

Depending on the intended purpose, in a further embodiment, it is possible that the PU matrix of the present invention additionally comprises further compounds. These further compounds, which preferably do not interact in a negative way with the PU matrix, add biological activities. Preferably, they are selected from the group consisting of Ca-salts; hydroxylapatite; small molecules, peptides or proteins having bone inducing properties; and small molecules, peptides, or proteins having anti-infective and/or anti-bacterial properties.

As set for the elsewhere herein, the content of free fatty acid is up to 19%, preferably between 7% and 19%, more preferably between 5% and 15% and most preferably between 7% and 10%, as determined by titration with sodium hydroxide These unreacted (free) fatty acids may build a kind of support or wall of the PU matrix. The reacted acids, such as the ricinoleic acid, may form a crosslinked structure that contributes to a hardness of the PU matrix. The calcium carbonate that can be added (which is then, after addition, present in the PU matrix) forms small spheres that are randomly distributed and have an osteoblast-attracting function, due to the increase in local alkaline phosphatase, which stimulates the presence of these osteoprogenitor cells.

In the present invention, it is hypothesized that at least one of these compounds contributes to a significant reduced antigen-antibody reaction.

The PU matrix of the present invention can be in the form of a liquid or a solid. The form of the PU matrix depends on the degree of polymerization of the matrix: The higher the degree of polymerization, the higher the rigidity or hardness of the PU matrix. The degree of liquidity (plasticity) can be varied (controlled) by changing the respective concentration of the ingredients. For example, a higher content of polyol, results in higher plasticity. As also disclosed elsewhere herein, during preparation of the PU matrix of the present invention, initially when the polyol component (also referred to herein as "Ampoule A") is mixed with the prepolymer (also referred to herein as "Ampoule B"), the resulting mixture is turning creamy during mixing, and then reaching plastic modelling and rubber state. After full curing (polymerization), the PU matrix will reach its greatest hardness.

Dependent on the intended use and/or function of the PU matrix, it may be preferred that the matrix exhibits higher plasticity than the plasticity that is preferred in another intended use/function.

It is remarkable and surprising that during preparation of the PU matrix and this during the polymerization process, the temperature of the PU matrix does not get higher than 47°C, preferably as measured inside the container, preferably not higher than 42°C, preferably in a state when it is applied to the implant site, such as the bone. The temperature of the PU matrix is measured according to common practice. This, in turn, provides for the beneficial effect that the occurrence of inflammatory processes in, or other damages to, the recipient's body is reduced, preferably that no inflammation or other damages occur that would be based on the presence of the PU matrix. On the other hand, however, the PU matrix can be subjected to any conventionally used forms of sterilization without being damaged itself.

Moreover, amongst other properties, this minimal heat generation enables that polymerization of the PU matrix (or the degree of polymerization) can be carried out in wet tissue environment, such as *in vivo* at the intended site. Further, the *in situ* generated PU matrix does not dissolve in liquid media such as blood or saliva. This further contributes to the improved suitability of the PU matrix of the present invention in applications regarding bone, particularly in the field of dentistry and orthopaedics. And moreover, the PU matrix of the present invention, contrary to some prior art products, does not exhibit volume loss after re-ossification.

Thus, the PU matrix of the present invention is particularly suitable for use in the field of medicine, such as dentistry and orthopaedics.

For example, the beneficial properties of the inventive PU matrix, in particular the specifically adjustable plasticity, provide for immediate and predictive immobilisation of e.g., dental implants, thereby allowing for immediate functional loading. Further, the improved handling of the PU matrix allows for quick and reliable 3D-reconstruction of any bony defect, such as local augmentation, sinus lift, bone splitting, block grafting, or the like. Further, the improved gluing properties enable immediate fixation and gluing of bone fractures and autologous grafts, as well as extremities. Moreover, the PU matrix of the present invention further contributes to the preservation of osteoporosis and prevention of fractures by internal stabilisation of bones and vertebrae.

In the field of dentistry, the PU matrix provides for an improved 3D-reconstructive treatment of e.g., periimplantitis as well as refixation of completely mobile and lost suprastructures, as well as the reconstruction of periodontal defects.

**EP 4 537 861 A1**

In a preferred embodiment, the PU matrix of the present invention is in form of a solid biocompatible flexible sheet and can be thermoformed (i.e., the PL) matrix is thermoformable) into changed shape by being deformed under heat, without elastic memory/return into its original shape. It is further preferred that the solid biocompatible flexible sheet has a thickness in the range of from equal to or more than 0.01mm to equal to or less than 20.00mm. The thickness depends on the intended use of the PU matrix. For example, it is possible that the PU matrix has a thickness in the range of from 0.01mm to less than 1.00mm, and in this case, the PU matrix is referred to as membrane. It is also possible that the PU matrix has a thickness in the range of from 1.00mm to less than 5.00mm, and in this case, it is referred to as blade. And further, it is also possible that the PU matrix has a thickness being in the range of from 5.00mm to less than 20.00mm; in this case, it is referred to as block. A block of the PU matrix of the present invention is particularly suitable for bone segment machining. For example, a PU matrix being in form of a plate can be formatted using a heat source, and after formatting, there is no elastic memory present.

The present invention further refers to a process for preparing a PU matrix, preferably the PU matrix disclosed herein. This process comprises the following steps (a) to (e):

In a first step a), a polyol component (also referred to herein as "Ampoule A") is provided, with the polyol component exhibiting the following characteristics (a-i) and (a-ii):

(a-i) an acidity index being in the range of from 0.8-8.5 mgKOH/g, preferably from 1.0-7.5 mgKOH/g, more preferably from 1.5 to 7.0 mgKOH/g, even more preferably from 2.5-6.5 mgKOH/g, even more preferably from 4.0-5.5 mgKOH/g, and most preferably from 4.5-5.0 mgKOH/g, as determined by titration; and

(a-ii) a hydroxyl index in the range of from 200 to 450 mgKOH/g preferably from 250 to 400 mgKOH/g, more preferably from 300 to 400 mgKOH/g, and most preferably from 360 to 380, as determined by the method disclosed in "The International Pharmacopoeia - Eleventh Edition, 2022, 4.7 Determination of hydroxyl value".

Then, in step b), a diisocyanate component, preferably diphenylmethane-4,4'-diisocyanate (MDI), is provided, wherein the diisocyanate component exhibits the following characteristics (b-i) and optionally (b-ii):

(b-i) an amount of free isocyanate as NCO equivalent being in the range of from 16% to 35% preferably from 22% to 35%, more preferably from 25% to 32% as determined by addition of excess di-n-butyl amine to form a urea and subsequent back titration with hydrochloric acid of the unreacted amine.

Optionally (b-ii) a density being in the range of from 1.11 to 1.28 $g/cm^3$, as determined by pycnometry.

Then, in step c), a part of the polyol component of a) and a part of the diisocyanate component of b) is combined to form a prepolymer. This prepolymer is also referred to herein as "Prepolymer" and as "Ampoule B".

In a preferred embodiment, the formed prepolymer of step c) has an amount of free isocyanate as NCO equivalent being in the range of from 10% to 25%, preferably from 19% to 23%, more preferably from 20 to 21%, as determined by addition of excess di-n-butyl amine to form a urea and subsequent back titration with hydrochloric acid of the unreacted amine, a density that is in the range of from 1000 to 1500 $g/cm^3$, preferably from 1100 to 1400 $g/cm^3$, more preferably from 1180 to 1270 $g/cm^3$, determined as described elsewhere herein; and/or, preferably and, a dynamic viscosity being in the range from 350-750 centipoise (cP), preferably from 400 to 700, more preferably from 450 to 650 cP, and even more preferably from 500 to 600 cP, as determined with a rotational viscometer by American Standards of Testing and Materials ASTM D4878: Standard Test Methods for Polyurethane Raw Materials: Determination of Viscosity of Polyols, Volume 08.02 (DOI: 10.1520/D4878-15) valid at the effective date.

It is additionally preferred that in step c), the following parts of polyol component and diisocyanate component are combined: 1 part of polyol and 1 to 1.7 part of diisocyanate; preferably 1 part of polyol to 1.2 to 1.6 part of diisocyanate, and more preferably 1 part of polyol to 1.3 to 1.5 part of diisocyanate. In a preferred embodiment, by combining the parts of polyol component and diisocyanate component as indicated above, the diisocyanate component is present in the prepolymer in an amount of 20% to 32%.

Then, in step d), at least a part of the polyol component of a) and at least a part of the prepolymer of c) is combined, thereby arriving at a mixture. In a preferred embodiment, in step d), the ratio of the prepolymer of step c) that is combined with the polyol of step a) to obtain a polyurethane matrix is in a range of from 0.5:1.0 to 0.8:1.0 (polyol/prepolymer).

Then, in a final step e), the PU matrix is obtained.

In a further preferred embodiment, the polyol component (a) additionally has

(a-iii) a density being in the range from 850-1200 $g/cm^3$, preferably from 950 to 1200 $g/cm^3$, more preferably from 1000 to 1150 $g/cm^3$ , and most preferably from 1050 to 1100 $g/cm^3$, respectively as determined by pycnometry at a temperature of 25°C; and

(a-iv) a viscosity being in the range from 1400 to 1800 cP, preferably from 1500 to 1700 cP, as determined by ASTM D4878, Volume 08.02 (DOI: 10.1520/D4878-15) valid at the effective date.

b) the diisocyanate component (b) additionally has

(b-iii) a water content of equal to or less than 0.1%, preferably being in the range from 0.01 % to 0.1%, more preferably the water content is 0%, as determined by moisture content (oven dry method); and

(b-iv) a viscosity being in the range from 20 cP to 200 cP, preferably being in the range from 20 cP to 120 cP, more preferably being in the range from 40 cP to 120 cP, and even more preferably being in the range from 40 cP to 100 cP, as determined by ASTM D4878, Volume 08.02 (DOI: 10.1520/D4878-15) valid at the effective date.

In the process of the present invention, the polyol component of step a) is prepared from castor oil that has the following properties:

- an acidity index being in the range of from 0.20-1.5 mgKOH/g, preferably from 0.40-1.25 mgKOH/g, more preferably from 0.5 to 1.0 mgKOH/g, determined as disclosed elsewhere herein;
- a hydroxyl index in the range of from 150-170, preferably from 150-160, more preferably from 155 to 165, determined as disclosed elsewhere herein; and optionally
- a density being in the range from 0.090-0.098 g/cm$^3$, preferably from 0.095-0.096 g/cm$^3$, as determined by pycnometry at a temperature of 25°C; and/or
- a viscosity being in the range from 500 cP to 1000 cP, preferably being in the range from 600 cP to 900 cP, more preferably being in the range from 700 cP to 800 cP, determined as disclosed elsewhere herein.

In a preferred embodiment, the polyol component of step a) is prepared from castor oil having all of the above defined properties with regard to acidity index, hydroxyl index, density, and viscosity.

When the above conditions and ranges are observed in the PU matrix preparation process, the PU matrix disclosed herein can be achieved. Suitable further conditions and process embodiments are described in further detail below.

In step a), a polyol component is provided. In a preferred embodiment, this step comprises step (a-1) of combining castor oil, ethylene glycol and propylene glycol under protective atmosphere, preferably nitrogen atmosphere; preferably, the combining is carried out under stirring; preferably for a time period of 1 to 5 hours, more preferably of 2 to 4 hours, even more preferably for about 2 hours; thereby arriving at a mixture; step (a-2) of heating up the mixture of (a-1), preferably heating up to a temperature in the range of from about 100°C to about 270°C, more preferably to a temperature in the range from about 200°C to about 260°C, even more preferably to a temperature of about 250°C, followed by a step of cooling; an optional step (a-3) of rotating the mixture, preferably under reduced pressure, preferably in a rotary evaporator, thereby eliminating residual gas; an optional step (a-4) of cooling the mixture; and step (a-5) of obtaining the polyol component. In a more preferred embodiment, step a) comprises all steps (a-1) to (a-5).

In step c), a prepolymer is formed by combining a part of the polyol component a) and a part of the diisocyanate component of b). In a preferred embodiment, this step c) comprises the following steps (c-1) to (c-5):

In step (c-1), the polyol of step (a) is mixed with a diisocyanate compound (preferably diphenylmethane-4,4"-diisocyanate (MDI)), preferably in a ratio as defined above (that is, the following parts of polyol component and diisocyanate component are combined: 1 part of polyol and 1 to 1.7 part of diisocyanate; preferably 1 part of polyol to 1.2 to 1.6 part of diisocyanate, and more preferably 1 part of polyol to 1.3 to 1.5 part of diisocyanate), thereby obtaining a mixture. In step (c-2), the mixture of (c-1) is stirred, preferably at a temperature of about 100°C. In an optional step (c-3), a vacuum is applied. In step (c-4), possible gas inclusion is removed, preferably by evaporating the mixture; and finally, in step (c-5), the prepolymer is obtained.

It is possible that the process of the present invention comprises a step of adding further compounds. These further components, which preferably do not negatively influence the properties of the PU matrix of the present invention, are selected from the group consisting of Ca-salts, such as $CaCO_3$ or $CaCO_3(PO_4)_2$, wherein preferably these salts are present in a total amount of 10wt% to 55wt%, preferably 20wt% to 50wt%, more preferably 30wt% to 50wt%, even more preferably 30wt% to 45wt%, and most preferred in a total amount of 30wt% to 40wt%, based on the sum of the weight of the main components polyol and prepolymer; hydroxylapatite, wherein preferably the hydroxylapatite is present in an amount of about 15-25wt%, more preferably in an amount of 18-22wt.%, and most preferably in an amount of about 20wt.%, based on the sum of the weight of the main components polyol and prepolymer; small molecules, peptides, or proteins having bone inducing properties, wherein preferably the total amount of said components is in a range of from about 1wt% to about 5wt%, based on the sum of the weight of the main components polyol and prepolymer; and small molecules, peptides, or proteins having antiinfective and/or antibacterial properties, in a suitable amount.

By the action of the Ca-salts, hydroxylapatite, and the small molecules, peptides, or proteins, osseoconduction and/or, preferably and, osseoregeneration is induced, and antiinfective/antibacterial properties are conferred.

In a preferred embodiment, the further compound that is added in a further step is a Ca-salt such as $CaCO_3$ or $CaCO_3(PO_4)_2$, preferably the further compound that is added is $CaCO_3$. The $CaCO_3$ provides for small spheres in the PU matrix that are randomly distributed. These spheres are able to attract osteoblasts, finally stimulating the presence of osteoprogenitor cells which, in turn, result in the generation of new bone.

The further component can be added during the process of the present invention during any suitable step. Preferably, the further component is added during or after step d), preferably, the further compound is added during or after step d), but prior to step e). More preferred, the further compound is added during step d), thereby being part of the mixture of step d).

In step e), the PU matrix is obtained. In a preferred embodiment, this step of obtaining the PU matrix comprises a step of polymerizing the mixture of step d), i.e., the mixture that is obtained when combining at least a part of the polyol component and a part of the prepolymer. The polymerization is initiated by mixing (combining) the polyol component ("Ampoule A") and prepolymer ("Ampoule B"). Depending on the ratio of the polyol component a) and the prepolymer, the degree and speed of polymerization can be controlled. For example: Ampoule A and Ampoule B are mixed in a ratio of 0.60-0.7 to 1.

During the polymerization reaction, the maximum polymerization temperature that is developed during the polymerizing step is at most 47°C, preferably at most 46°C, or at most 45°C, when measured inside a container where step d) takes place. In a further preferred embodiment, during step e), no polymerization catalyst is present. By control of the precise amounts of polyol and prepolymer containing isocyanates as indicated above the polymerisation reaction starts without further initiation in a controlled manner. This is advantageous e.g., in terms of toxicity, as no toxic reaction products and/or by-products are generated.

As shown below in the Examples the polymerization can take place *in vivo* as liquid or viscous prepolymer or *ex vivo* forming fixed implants or complex prosthetics applications. These characteristics will allow multiple application in the surgical setting which could not be performed so far. In addition, after the polymerization process is finished, the material does not have any volume change and thus avoid the problems observed with previous castor oil polymers such as Kryptonite (Doumit et al. 2014).

The polymer can also be produced as blocks, granules, membranes, plates, large prototyped bone segments and solid or flexible frameworks either alone or in combination with ceramics or metals. Moreover, bone frameworks for cell culture and proliferation can be produced with the polymer to create a suitable environment and to produce autogenous tissues grafts, using the patients stems cells to repair major bone defects.

Depending on the chosen conditions the surface of this biocompatible polyurethane can be smooth or rough and its interior can contain numerous interchangeable canaliculi, susceptible to penetration of the cellular stroma. These physical characteristics help in stability, anchoring and accelerating the new bone replacement. The polymer can be also modified by addition of Ca-salts such as Ca-carbonate or Ca-carbonate phosphate.

The high biocompatibility is also attributable to the observed inhibition of microbiological growth and formation of biofilms, a major thread for bone implants and prothesis (Miwa et al. 2019). This is achieved either by the polymer itself and/or combination with suitable anti-microbial agents.

The present invention further relates to a PU matrix, obtainable or obtained by the process as defined herein. This obtained PU matrix exhibits one or more, preferably all, of the advantages listed herein.

Due to the beneficial properties of the PU matrix of the present invention, it is very well suitable for being used in the field of medicine, such as in dentistry or orthopedics. Particularly, the present invention therefore refers to the PU matrix for use in a method of bone regeneration, bone fixation, bone adhesion ("glue"), bone implantation, and substitution of a whole or a part of a bone. For example, the PU matrix can be used as a glue, e.g., in bone bonding in bone fractures in external bone cerclage.

In a preferred embodiment, the method comprises comprises preparing implants such as dental implants or orthopaedic implants. In a preferred embodiment, said implants are individualized implants. Individualized implants are particularly beneficial if these implants are to be adjusted to a specific subject (patient) and therefore can only be used for this specific subject, in other words, if these implants are individualized implants. Such individualized implants are for example dental implants, or other bone implants where it is to be preferred to individualize it, such as implants for vertebrae, or for the skull.

It is also possible that the PU matrix of the present invention is used for stabilizing bone structure.

Examples of possible uses of the PU matrix are in orbital floor replacement. Further examples for custom prototyped prostheses are in the field of cranioplasty, such as skull bone replacement.

It is also possible to "preform" the PU matrix of the present invention into a desired shape (form) using an external heat source, and to make the final adjustments of the prosthesis.

The PU matrix of the present invention can also be used in tumor surgery, for example a part of a jaw can be replaced by a corresponding part that is formed from the PU matrix of the present invention.

In a further embodiment, the PU matrix is provided subcutaneously to a subject.

Definitions

Within the meaning of the present invention, the term "free fatty acids" refers to fatty acids that are not esterified and may be unbound, i.e., not bound to a protein.

Methods

*Determination of the acidity index*

The acidity index (also referred to as "acid value") is determined by titration. In detail, the amount of free fatty acids that are present in a substance is measured by its acid value and called as acid value in fats and oils. It is expressed as the amount of potassium hydroxide (KOH) required to neutralize the free fatty acids present in one gram of the substance (or sample, respectively). In the present invention, the acidity index is determined by carrying out a titration, e.g. as described in Sarmila K. C.,"Determination of Acid Value in Fats and Oils", The Science Notes, Simplest Explanations of Complex Facts", April 12, 2023.

*Determination of hydroxyl index*

The hydroxyl index (also referred to as "hydroxyl value") is determined as described in "The International Pharmacopoeia - Eleventh Edition, 2022, 4.7 Determination of hydroxyl value"Method A.

*Determination of free isocyanate*

The free isocyanate (NCO) is determined by reaction of NCO with di-n-butyl amine to form a urea. Unreacted (excess) amine is determined by back titration with hydrochloric acid according to ISO 14896, in the version July 2009 valid at the effective date, e.g. described in https://www.iso.org/standard/50601.html.

*Determination of density of polyol*

The density of the polyol is measured using a pycnometer according to American Standards for Testing and Materials ASTM D4669, Determination of Specific Gravity of Polyols, Volume 08.02, DOI: 10.1520/D4669-18, https://www.astm.org/d4669-18.html

*Determination of water content*

The water content e.g., of diisocyanate, is preferably determined by moisture content method (oven dry method).

*Determination of content of free fatty acids*

The amount of free fatty acid in % is determined according to the American Oil Chemists' Society AOCS Ca 5a-40, reapproved 2009 (described in https://www.scribd.com/document/501700643/AOCS-Ca-5a-40#). The free fatty acid is determined by titrating the sample in ethanol with standard aqueous sodium hydroxide using a phenolphthalein indicator solution.

*Equivalent weight (Adapted from a Huntsman Technical Center Bulletin)*

The equivalent eight (eq.wt.) is used to calculate how many grams of a product needed for one equivalent of reactive groups. E.g., for an isocyanate, the reactive group is NCO (N=C=O). Its concentration is measured by weight percent NCO.

$$\text{Isocyanate equivalent weight} = \frac{4.200}{NCO}\, g/eq$$

*Example:*

$$\text{Isocyanate eq. wt. of pure MDI} = 4200 \div 33.6 = 125\ g/eq$$

For a polyol, the reactive group is -O-H (OH).
The OH concentration is measured by the OH value (mg KOH/g sample):
Equivalent weight of a polyol = 56100 ÷ OH Value (unit: g/eq)

*Example:*

$$\text{Eq. wt. of ethylene glycol} = 56100 \div 1810 = 31.0 \text{ g/eq}$$

*Molecular weight of a polyol (Adapted from a Huntsman Technical Center Bulletin):*

The molecular weight (mol. wt.) of a polyol is approximated by the polyol equivalent weight (eq. wt.) multiplied by the nominal functionality (fn):

$$\text{Polyol mol. wt.} = (\text{eq.wt.}) \times (\text{fn}) = [56100 \div \text{OH value}] \times (\text{fn}) \text{ (unit: g/mol)}$$

$$\text{Mol. wt. of any diol} = [56100 \div \text{OH value}] \times 2 \text{ g/mol}$$

$$\text{Mol. wt. of any triol} = [56100 \div \text{OH value}] \times 3 \text{ g/mol}$$

*Example:*

$$\text{Mol. wt. of ethylene glycol} = [56100 \div 1810] \times 2 = 62.0 \text{ g/mol}$$

*Total weight of MDI (Diphenylmethane Diisocyanate) required for reaction (Adapted from a Huntsman Technical Center Bulletin):*

When reacting an isocyanate with one or more polyols to form a polyurethane, one NCO group reacts with one OH group. When the number of NCO groups equals the number of OH groups, you have a stoichiometric NCO : OH ratio of 1.0. This ratio is commonly referred to as the index. To determine the amount of MDI required to react with a given polyol blend, you must know the desired index (often 1.0), the MDI equivalent weight (MDI eq. wt.), and the weight fractions (pbw) and equivalent weights of the polyols and any water present in the blend.

$$\text{Total weight of MDI required} =$$
$$\text{index} \times \text{MDI eq. wt.} \times \{(\text{pbw polyol A} \div \text{eq. wt. polyol A}) + (\text{pbw polyol B} \div \text{eq. wt. polyol B})$$
$$+ \ldots\ldots + (\text{pbw polyol N} \div \text{eq. wt. polyol N}) + (\text{pbw water} \div \text{eq. wt. water})$$

*Example formulation:*

Given is the following polyol blend and one would like to determine the amount of pure MDI to add to achieve a fully reacted polyurethane with a slight excess of isocyanate (target index = 1.05)

| | |
|---|---|
| Polyol A (OH value = 490) | 100.00 |
| 1,4 Butanediol (BD) (eq. wt. = 45) | 20.00 |
| Water | 0.15 |
| pure MDI (% NCO = 33.6) | X |

$$X = \text{pbw MDI} = \text{index} \times \text{MDI eq. wt.} \times \{[100 \div (56100 \div 490)] + (20 \div 45) + (0.15 \div 9)\}$$

$$X = 1.05 \times (4200 \div 33.6) \times \{0.873 + 0.444 + 0.017\}$$

$$X = 1.05 \; x \; 125 \; x \; 1.334$$

$$X = 175 \text{ pbw MDI}$$

*Total amount of MDI required for reaction*

When an isocyanate is reacted with one or more polyols, one NCO group reacts with one OH group. When the number of NCO groups is equal is to the number of OH groups, the result is a stoichiometric NCO : OH ratio of 1.0. A ratio of 1.0 is commonly known as the index.

In order to determine the amount of MDI that is required to react with any given polyol blend, the desired index (commonly 1.0) must be known, as well as the MDI equivalent weight (MDI eq. wt.), the weight fractions (pbw) and the equivalent weights of the polyols and any water present.

Examples

**Example 1**

Polyol synthesis

Castor oil (e.g., EC / List no.: 232-292-2, CAS no.: 8001-78-3) is combined with ethylene glycol and propylene glycol (e.g., ratio Castor oil/polyethylene glycol/propylene glycol 1 :0.2/0.1), stirred under nitrogen atmosphere for 2 hours and heated up in a glass synthesis reactor to 250 °C. The distilled water is separated, and the mixture is cooled down. Subsequently the mixture is rotated at 120°C in a rotary evaporator eliminating residual gas. After cooling down the product (polyol) is stored in the dark at room temperature.

The polyol is analysed by color, hydroxyl index, density, and acidity. To be further used, the polyol is slightly yellow viscous transparent solution, and is selected to have a hydroxyl index of 200 to 450 mgKOH/g and an acidity index of 0.8-8.5 mgKOH/g.

For use in clinical setting the polyol is filled under sterile conditions into ampoules or syringes.

**Example 2**

Prepolymer synthesis

The polyol from Embodiment 1 is mixed with Diisocyanate (e.g., MDI 4,4- Diphenylmethane isocyanate RUBINATE 1680 from Bayer AG, Germany) in a ratio of 3:1 and stirred at 100°C. Possible inclusion of gas is removed by putting the liquid in an evaporator for approx. 15 min. The product is assessed by color, density, viscosity, and content of free isocyanate. The free isocyanate may range between 10% to 35%, and for further use is typically selected to be in the range from 20% to 32%, preferably from 22% to 30%, in particular from 28% to 29%.

**Example 3**

Augmentation of the polymer with $CaCO_3$

The polyol from Example 1 and the prepolymer from Example 2 were further modified by the addition $CaCO_3$. For this purpose, $CaCO_3$ powder (20g) was respectively mixed with the polyol and the prepolymer (total amount 40g) and gently stirred until the Ca-salt was homogeneously dispersed.

After mixing, the resulting viscous polymer was hardening over a period of 30 min to its final strength.

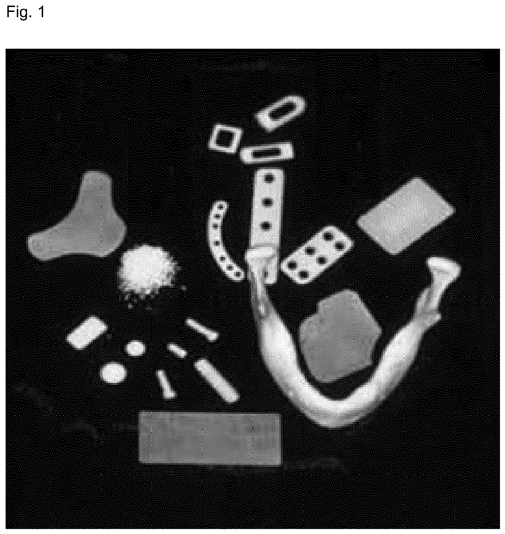

Fig. 1 shows various examples - e.g., implant forms of the final polymer (PU matrix).

**Example 4**

A PU polymer matrix was prepared by mixing and let polymerize the components prepared in Examples 1 and 2.

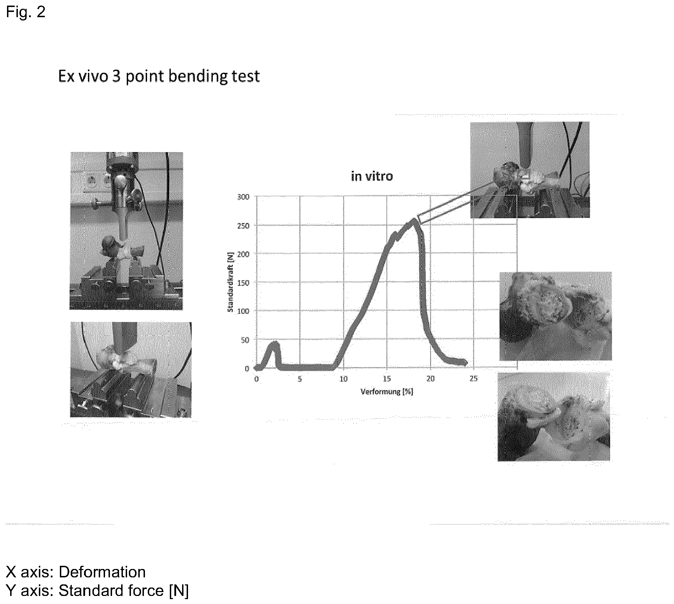

To assess the stability of the polymer prepared from the components prepared in Examples 1 and 2 as a bone glue, a mini pig femur bone was cut by a bone oscillating saw. Subsequently the two surfaces were exposed to the viscous polymer and the two bone fragments were hold in the native position (prior to the cut). After 15 min the glue hardened, and the bone fracture was stably connected. The bone was left at room temperature without any further fixation. The following day the bone and a control femur bone from the same animal were prepared for assessment of mechanical stability using a three-point bending stress device. As can be seen from Fig. 2, the bone has a high strength.

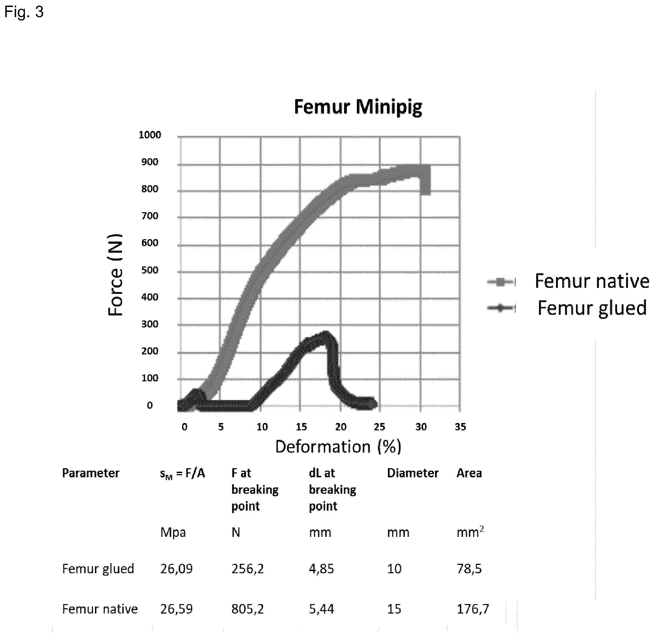

As compared to the native bone the strength was reduced threefold despite the small glue area. The strength of the glue is comparable to the native bone as indicated by the sM. (Fig. 3).

**Example 5**

Temperature profile during polymerization

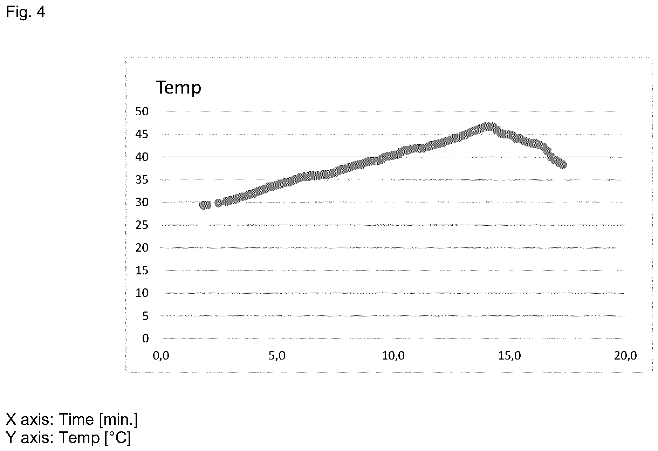

Polyol as described in the Example 1 and the prepolymer as described in Example 2 were mixed and stirred during the following 15 min until viscosity is achieved. In parallel the temperature in the mixture was determined. As can be seen in Fig. 4 the temperature curve has a peak of 46°C at 13 min. At this time the viscous material can be taken from the plastic form and used as glue or implant, cooling rapidly down within 5 min to temperatures below 39°C.

**Example 6**

The osseointegrative properties of a polymer implant was assessed in a rabbit model of a radius bone defect. Under anesthesia the rabbit radius bone was prepared, and a portion of the radius was removed and an implant as prepared according to Examples 1 to 3 was inserted. It was observed that the implant was completely integrated into the radius bone and gradually replaced by bone tissue.

Reference list

M.P. Mandarino, J.E. Salvatore.
Polyurethane polymer; its use in fractured and diseased bones. Am J Surg. 1959 Apr;97(4):442-6. doi: 10.1016/0002-9610(59)90011-x. PMID: 13627384.

US Patent Application 2012/0265312 A1 Oct. 2012 System and Method for Manufacture of a Cranial Repair Implant and a Cranial Repair Implant Produced Thereby; Inventor Shawn Burke, Michael Teague, Pat Lemoyne

MR. Norton, GW. Kay, MC. Brown, DL. Cochran Bone glue - The final frontier for fracture repair and implantable device stabilization; Int. J. Adhesion and Adhesives; 102, 10264, 2020

Pieterjan Valvekens, Dirk De Vos Chapter 1 - Double Metal Cyanides as Heterogeneous Catalysts for Organic Reactions New Materials for Catalytic Applications 2016, 1-12 https://doi.org/10.1016/B978-0-444-63587-7.00001-9

M Musik, M Bartkowiak, E Milchert Advanced methods for hydroxylation of vegetable oils, unsaturated fatty acids and their alkyl esters Coatings, 12,13 2022 https://doi.org/10.3390/coatings12010013

N. L. Parada Hernandez, A. J. Bonona, J. O. Bahúa, M. I. R. Barbosaa, M. R. W.f Maciel, R. M. Filho Epoxy monomers obtained from castor oil using a toxicity-free catalytic system. J. of Molecular Catalysis A: Chemical 2017

J. Zhang, Y. Wu, H. Zhang, T. Yan, Y. Huang, J. Jiang, J. Tang Castor oil-glycerol-based waterborne polyurethane dispersions Progress in Organic Coatings, 157, 106333 2021

T. P. T. de Sousa, M. S. T. da Costa, R. Guilherme, W. Orcini, L. de Andrade Holgado, E. M. Varize Silveira, O. Tavano, A. G. Magdalena, S. A. Catanzaro-Guimarães, A. Kinoshita Polyurethane derived from Ricinus Communis as graft for bone defect treatments Polimeros, 28(3), 246-255, 2018

MM. Beloti, KN. Hiraki, VMR Barros, AL Rosa Effect of the chemical composition of Ricinus Communis polyurethane on rat bone marrow cell attachment proliferation, and differentiation Journal of Biomaterial Research 64, 171, 2003

G.D. Doumit, E. Meisler, J. Sidaoui, J.E. Zins, F.A. Papay The expansile properties of kryptonite relating to cranioplasty J Craniofac Surg. 2014 May; 25(3):880-3. doi: 10.1097/SCS.0000000000000508.

A. Palanisamy, M. S. L. Karuna, T. Satyavani, D. B. Rohini Kumar Development and Characterization of Water-Blown Polyurethane Foams from Diethanolamides of Karanja Oil J. American Oil Chemist's Society 88, 4, 541, 2011

S.,Miwa, T. Shirai, N. Yamamoto, K. Hayashi, A. Takeuchi, K. Tada,Y. Kajino, T. Higuchi, K. Abe, H. Aiba, Y. Taniguchi, H. Tsuchiya. Risk factors for surgical site infection after malignant bone tumor resection and reconstruction. BMC Cancer. 2019 Jan 8;19(1):33. doi: 10.1186/s12885-019-5270-8. PMID: 30621654; PMCID: PMC6325841.

Hatt LP, Thompson K., Helms J.A., Stoddart M.J., Armiento A.R. Clinically relevant preclinical animal models for testing novel cranio-maxillofacial bone 3D-printed biomaterials; Clin. Transl. Med. (2022),12:e690

**Claims**

1. Polyurethane (PU) matrix, wherein

   i) a water content of the PU matrix is equal to or less than 1%, optionally equal to or less than 0.1%, or there is no water present, as determined by moisture content measurement method (oven dry method); and
   ii) free fatty acid is present but the content thereof is up to 19 %, as determined by titration with sodium hydroxide,

   with the respective properties being determined at a temperature of 25°C.

2. PU matrix according to claim 1, wherein the matrix additionally exhibits one or more,

   preferably all, of the following properties:

   iii) physical resistance capacity against traction being in the range of between 26 MPa and 42 MPa; preferably being in the range of between 30 MPa and 38 MPa, as determined according American Society of Testing and Materials ASTM D695MM;
   iv) elastic modulus being in the range of between 1700 Mpa and 2400 Mpa, preferably being in the range of between 1900 Mpa and 2200 Mpa, as determined according to American Society of Testing and Materials ASTM D695MM;
   v) compression being in the range of between 48 Mpa and 57 Mpa, preferably in the range of between 50 Mpa and 55 Mpa, as determined according to American Society of Testing and Materials ASTM D695MM;
   vi) deformation being in the range of between 3.7% and 5.5%, preferably in the range of between 4.0% and 5.0%, as determined according to American Society of Testing and Materials ASTM D695MM; and
   vii) hardness shore D being in the range between 72 and 84, preferably between 75 and 80, as determined by a durometer according to American Standards for Testing and Materials ASTM D2240,

   with the respective properties being determined at a temperature of 25°C

3. PU matrix according to claim 1 or 2, additionally comprising further compounds being selected from the group consisting of Ca-salts; hydroxylapatite; small molecules, peptides or proteins having bone inducing properties; and small molecules, peptides, or proteins having antiinfective and/or antibacterial properties.

4. Process for preparing a polyurethane matrix, comprising the following steps:

   a) providing a polyol component, wherein the polyol component has:

   (a-i) an acidity index being in the range of from 0.8-8.5 mgKOH/g, preferably from 1.0-7.5 mgKOH/g, more preferably from 1.5 to 7.0 mgKOH/g, even more preferably from 2.5-6.5 mgKOH/g, even more preferably from 4.0-5.5 mgKOH/g, and most preferably from 4.5-5.0 mgKOH/g, as determined by titration;
   (a-ii) a hydroxyl index in the range of from 200 to 450 mgKOH/g preferably from 250 to 400 mgKOH/g , more preferably from 300 to 400 mgKOH/g, and most preferably from 360 to 380, as determined by the method disclosed in "The International Pharmacopoeia - Eleventh Edition, 2022, 4.7 Determination of hydroxyl value"; and

   b) providing a diisocyanate component, preferably diphenylmethane-4,4'-diisocyanate (MDI), wherein the diisocyanate component has:

   (b-i) an amount of free isocyanate as NCO equivalent being in the range of from 16% to 35%, preferably from 22% to 35%, more preferably from 25% to 32% as determined by addition of excess di-n-butyl amine to form a urea and subsequent back titration with hydrochloric acid of the unreacted amine;
(b-ii) optionally a density being in the range of from 1.11 to 1.28 g/cm$^3$, as determined by pycnometry;

c) combining at least a part of the polyol component of a) and at least a part of the diisocyanate component of b) to form a prepolymer;
d) combining at least a part of the polyol component of a) and at least a part of the prepolymer of c), thereby preparing a mixture; and
e) obtaining the polyurethane matrix from said mixture.

5. Process according to claim 4, wherein in step c) the following parts of polyol

component and diisocyanate component are combined: 1 part of polyol and 1 to 1.7 part of diisocyanate; preferably 1 part of polyol to 1.2 to 1.6 part of diisocyanate, and more preferably 1 part of polyol to 1.3 to 1.5 part of diisocyanate.
and/or
wherein in step d), the ratio of the prepolymer of step c) that is combined with the polyol of step a) to obtain a polyurethane mixture is in a range of from 0.5:1.0 to 0.8:1.0 (polyol/prepolymer).

6. Process according to claim 4 or 5, comprising a further step of adding further compounds, wherein the further compounds are selected from the group consisting of

• Ca-salts such as $CaCO_3$ or $CaCO_3(PO_4)_2$, wherein preferably these salts are present in a total amount of 10wt% to 55wt%, preferably 20wt% to 50wt%, more preferably 30wt% to 50wt%, even more preferably 30wt% to 45wt%, and most preferred in a total amount of 30wt% to 40wt%, based on the sum of the weight of the polyol and prepolymer;
• hydroxylapatite, wherein preferably the hydroxylapatite is present in an amount of about 15-25wt%, more preferably in an amount of 18-22wt.%, and most preferably in an amount of about 20wt.%, based on the sum of the weight of the polyol and prepolymer;
• small molecules, peptides, or proteins having bone inducing properties, wherein preferably the total amount of said components is in a range of from about 1wt% to about 5wt%, based on the sum of the weight of the polyol and prepolymer; and
• small molecules, peptides, or proteins having antiinfective and/or antibacterial properties, in a suitable amount.

7. Process according to any one of claims 4 to 6, wherein step e) comprises a step of polymerizing the mixture of step d).

8. Process according to any one of claims 4 to 7, wherein during step e) no polymerization catalyst is present.

9. PU matrix, obtainable by the process as defined in any one of claims 4 to 8.

10. The PU matrix according to any one of claims 1 to 3, or according to claim 9, for use in a method of therapeutic treatment for bone regeneration, bone fixation, bone adhesion, bone implantation, and/or substitution of a whole or a part of a bone.

11. The PU matrix for use according to claim 10, wherein the method of treatment comprises preparing implants, preferably dental implants or orthopaedic implants, more preferably said implants are individualized implants, in particular orthopaedics or dental individualized implants for use in preparing dental implants, more preferably for use in preparing individualized dental implants.

12. The PU matrix for use according to claim 10 or 11, wherein the implants are suitable for replacing bones; for replacing parts of bones; for replacing bone defects; or for stabilizing bone structures.

13. The PU matrix for use according to any one of claims 10 to 12, wherein the PU matrix is provided to a subject subcutaneously.

14. A kit comprising the polyol component as defined in claim 4, and the prepolymer as defined in claims 4 or 5.

15. The kit according to claim 13 or 14, further comprising one or more of the compounds selected from the group consisting of Ca-salts, hydroxylapatite, and small molecules, peptides, or proteins.

Fig. 1

Fig. 2

Ex vivo 3 point bending test

X axis: Deformation
Y axis: Standard force [N]

Fig. 3

**Femur Minipig**

Femur native
Femur glued

| Parameter | $s_M = F/A$ | F at breaking point | dL at breaking point | Diameter | Area |
|---|---|---|---|---|---|
| | Mpa | N | mm | mm | mm$^2$ |
| Femur glued | 26,09 | 256,2 | 4,85 | 10 | 78,5 |
| Femur native | 26,59 | 805,2 | 5,44 | 15 | 176,7 |

Fig. 4

X axis: Time [min.]
Y axis: Temp [°C]

**EUROPEAN SEARCH REPORT**

Europäisches
Patentamt

European
Patent Office

Office européen
des brevets

Application Number

EP 23 20 3649

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2016/298009 A1 (DEY TANMOY [US] ET AL) 13 October 2016 (2016-10-13) * claim 1 * * page 11 - page 15; examples 1-13 * | 1-15 | INV. A61L27/18 C08G71/04 |
| A | US 2011/236501 A1 (GUELCHER SCOTT A [US] ET AL) 29 September 2011 (2011-09-29) * claim 1 * * examples 1, 2 * | 1-15 | |
| X | DE MORAIS JOÃO PEDRO PEREIRA ET AL: "Polyurethane derived from castor oil monoacylglyceride (Ricinus communis) for bone defects reconstruction: characterization and in vivo testing", JOURNAL OF MATERIALS SCIENCE: MATERIALS IN MEDICINE, SPRINGER US, NEW YORK, vol. 32, no. 4, 1 April 2021 (2021-04-01), XP037431109, ISSN: 0957-4530, DOI: 10.1007/S10856-021-06511-Z [retrieved on 2021-04-01] * abstract * * page 3, paragraph 2.2 * | 1-15 | |
| A | TROVATI GRAZIELLA ET AL: "Characterization of polyurethane resins by FTIR, TGA, and XRD", JOURNAL OF APPLIED POLYMER SCIENCE, vol. 115, no. 1, 27 August 2009 (2009-08-27), pages 263-268, XP093142445, US ISSN: 0021-8995, DOI: 10.1002/app.31096 * abstract * * paragraph "Materials"; page 263 - page 264 * | 1-15 | |

TECHNICAL FIELDS SEARCHED (IPC)

A61L
C08G

The present search report has been drawn up for all claims

| Place of search | Date of completion of the search | Examiner |
|---|---|---|
| The Hague | 18 March 2024 | Guazzelli, Giuditta |

EPO FORM 1503 03.82 (P04C01)

## ANNEX TO THE EUROPEAN SEARCH REPORT
## ON EUROPEAN PATENT APPLICATION NO.

EP 23 20 3649

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2024

| Patent document cited in search report | Publication date | Patent family member(s) | | Publication date |
|---|---|---|---|---|
| US 2016298009 A1 | 13-10-2016 | BR | 112017021652 A2 | 10-07-2018 |
| | | CA | 2982120 A1 | 13-10-2016 |
| | | CN | 107847878 A | 27-03-2018 |
| | | DK | 3280516 T3 | 23-03-2020 |
| | | EP | 3280516 A1 | 14-02-2018 |
| | | HU | E048985 T2 | 28-09-2020 |
| | | JP | 6711503 B2 | 17-06-2020 |
| | | JP | 2018517009 A | 28-06-2018 |
| | | KR | 20170138468 A | 15-12-2017 |
| | | PL | 3280516 T3 | 01-06-2020 |
| | | TW | 201708295 A | 01-03-2017 |
| | | TW | 201940543 A | 16-10-2019 |
| | | US | 2016298009 A1 | 13-10-2016 |
| | | WO | 2016164526 A1 | 13-10-2016 |
| US 2011236501 A1 | 29-09-2011 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120265312 A1, Norton
- US 4990586 A
- US 20120265312 A, Inventor Shawn Burke, Michael Teague, Pat Lemoyne

### Non-patent literature cited in the description

- Determination of hydroxyl value. The International Pharmacopoeia. 2022, vol. 4
- **SARMILA K. C.** Determination of Acid Value in Fats and Oils. *The Science Notes, Simplest Explanations of Complex Facts*, 12 April 2023
- *Determination of Specific Gravity of Polyols*, vol. 08.02, https://www.astm.org/d4669-18.html
- *CHEMICAL ABSTRACTS*, 8001-78-3
- **M.P. MANDARINO, J.E. SALVATORE.** Polyurethane polymer; its use in fractured and diseased bones. *Am J Surg.*, April 1959, vol. 97 (4), 442-6
- **MR. NORTON ; GW. KAY ; MC. BROWN ; DL. COCHRAN.** Bone glue - The final frontier for fracture repair and implantable device stabilization. *Int. J. Adhesion and Adhesives*, 2020, vol. 102, 10264
- **PIETERJAN VALVEKENS ; DIRK DE VOS.** Double Metal Cyanides as Heterogeneous Catalysts for Organic Reactions New Materials for Catalytic Applications. 2016, 1-12
- **M MUSIK ; M BARTKOWIAK ; E MILCHERT.** *Advanced methods for hydroxylation of vegetable oils, unsaturated fatty acids and their alkyl esters Coatings*, 2022, vol. 12, 13, https://doi.org/10.3390/-coatings12010013
- **N. L. PARADA HERNANDEZ ; A. J. BONONA ; J. O. BAHÚA ; M. I. R. BARBOSAA ; M. R. W.F MACIEL ; R. M. FILHO.** Epoxy monomers obtained from castor oil using a toxicity-free catalytic system. *J. of Molecular Catalysis A: Chemical*, 2017
- **J. ZHANG ; Y. WU ; H. ZHANG ; T. YAN ; Y. HUANG ; J. JIANG ; J. TANG.** Castor oil-glycerol-based waterborne polyurethane dispersions. *Progress in Organic Coatings*, 2021, vol. 157, 106333
- **T. P. T. DE SOUSA ; M. S. T. DA COSTA ; R. GUILHERME ; W. ORCINI ; L. DE ANDRADE HOLGADO ; E. M. VARIZE SILVEIRA ; O. TAVANO ; A. G. MAGDALENA ; S. A. CATANZARO-GUI-MARÃES ; A. KINOSHITA.** *Polyurethane derived from Ricinus Communis as graft for bone defect treatments Polimeros*, 2018, vol. 28 (3), 246-255
- **MM. BELOTI ; KN. HIRAKI ; VMR BARROS ; AL ROSA.** Effect of the chemical composition of Ricinus Communis polyurethane on rat bone marrow cell attachment proliferation, and differentiation. *Journal of Biomaterial Research*, 2003, vol. 64, 171
- **G.D. DOUMIT ; E. MEISLER ; J. SIDAOUI ; J.E. ZINS ; F.A. PAPAY.** The expansile properties of kryptonite relating to cranioplasty. *J Craniofac Surg.*, May 2014, vol. 25 (3), 880-3
- **A. PALANISAMY ; M. S. L. KARUNA ; T. SATYA-VANI ; D. B. ROHINI KUMAR.** Development and Characterization of Water-Blown Polyurethane Foams from Diethanolamides of Karanja Oil. *J. American Oil Chemist's Society*, 2011, vol. 88 (4), 541
- **S.,MIWA ; T. SHIRAI ; N. YAMAMOTO ; K. HAYASHI ; A. TAKEUCHI ; K. TADA ; Y. KAJINO ; T. HIGUCHI ; K. ABE ; H. AIBA.** Risk factors for surgical site infection after malignant bone tumor resection and reconstruction. *BMC Cancer.*, 08 January 2019, vol. 19 (1), 33
- **HATT LP ; THOMPSON K. ; HELMS J.A. ; STOD-DART M.J. ; ARMIENTO A.R.** Clinically relevant preclinical animal models for testing novel cranio-maxillofacial bone 3D-printed biomaterials. *Clin. Transl. Med.*, 2022, vol. 12, e690